# EUROPEAN PATENT APPLICATION

(11) **EP 2 090 316 A1**
(43) Date of publication of application: **19.08.2009**
(21) Application number: 09250318.4
(22) Date of filing: 09.02.2009
(51) Int. Cl.: A61K 36/732, A61P 17/06

(54) **Cosmetic and dermatological composition for psoriatic skin treatment**

(30) Priority: 18.02.2008 IL 18956908
(71) Applicant: Levi-Hamdi, Eitan, Jerusalem 97792 (IL); Cohen, Sima, Pisgat Zeev 91443 (IL)
(72) Inventor: Faran, Mina, Pisgat Zeev 91443 (IL); Levi-Hamdi, Eitan, Jerusalem 97792 (IL)
(74) Representative: White, Duncan Rohan

(57) **Abstract**

Cosmetic and dermatological composition for the treatment of psoriatic skin, comprising 5% - 15% quince seeds; 10% - 40% natural base cream; and 55% - 75% mixture of anti-oxidants, skin softening agents, absorption aids, tissue regenerating and protecting agents, preservatives and thickening agents.

## Description

### FIELD OF THE INVENTION

The invention relates to cosmetic and dermatological composition for the treatment of psoriatic skin.

### BACKGROUND OF THE INVENTION

Psoriasis is a chronic disease which affects the skin and joints. It commonly causes red scaly patches to appear on the skin. The scaly patches caused by psoriasis, called psoriatic plaques, are areas of inflammation and excessive skin production. Skin rapidly accumulates at these sites and takes a silvery-white appearance. Plaques frequently occur on the skin of the elbows and knees, but can affect any area including the scalp and genitals. Psoriasis is believed to result from the body's immune system and is not contagious.

Psoriasis is a lifelong condition. There is currently no cure but various treatments can help to control the symptoms. Among the cures currently used are:
- Topical treatment, using bath solutions and moisturizers to help soothe affected skin and reduce the dryness which accompanies the build-up of skin on psoriatic plaques;
- Phototherapy, using daily, short, non-burning exposure to sunlight to helped clear or improve psoriasis;
- Photochemotherapy, combining the oral or topical administration of psoralen with exposure to ultraviolet A (UVA) light;
- Medications, taken internally by pill or injection; and others.

Published Patent Application WO2001066079 suggests a cosmetic and dermatological composition for psoriatic skin, scalp treatment and care.

### SUMMARY OF THE INVENTION

The present invention provides a cosmetic and dermatological composition for the treatment of psoriatic skin, to help soothe affected skin and reduce the dryness which accompanies the build-up of skin on psoriatic plaques.

The composition comprises 5% - 15% quince seeds; 10% - 40% natural base cream; and 55% - 75% mixture of anti-oxidants, skin softening agents, absorption aids, tissue regenerating and protecting agents, preservatives and thickening agents.

A method of preparing the composition is disclosed.

### DETAILED DESCRIPTION OF PREFERRED EMBODIMENTS

The present invention provides a cosmetic and dermatological composition for the treatment of psoriatic skin, to help soothe affected skin and reduce the dryness which accompanies the build-up of skin on psoriatic plaques.

The composition according to the present invention contains (weight percents): 5% - 15% quince seeds jelly - the seeds, soaked or boiled in water, release the mucilage from the seed coat and make a jelly-like consistency into which the remaining ingredients are stirred. In an alternative embodiment, Linseed (flax) may be used in a similar fashion;
10% - 40% natural base cream, such as Ferntree Cottage Pure Base cream made at Ferntree Cottage, Ireland, Essential Base Cream, available from Essential Therapeutics, Hallam, Austalia, or any other commercially available natural base cream;
55% - 75% mixture of anti-oxidants, skin softening agents, absorption aids, tissue regenerating and protecting agents, preservatives and thickening agents.

Anti-oxidants may include Vitamin E, wheat germ oil, Safflower oil, or any other natural anti-oxidant known in the art.

Skin softening agents may include sweet almond oil, sesame oil, or any other natural skin softening agent known in the art.

Absorption aids may include jojoba oil, vegetable squalene, or any other natural absorption aid known in the art.

Tissue regenerating and protecting agents may include grape seed oil, sunflower oil, or any other natural skin tissue regenerating and protecting agents known in the art.

Preservatives may include paraben, tea tree essential oil, thyme essential oil, grapefruit seed extract, D-Alpha Tocopherol Acetate (Vitamin E), or any other natural preservative known in the art.

Thickening agents may include bee wax, aloevera, medicinal vaseline, coconut oil, guar gum, palm oil, borax, or any other natural thickening agent known in the art.

According to a preferred embodiment of the present invention, the quince seeds jelly comprises 10% by weight of the composition, the base cream comprises 20% by weight of the composition and the remaining 70% mixture comprises:
Anti-oxidants - 2% vitamin E and 2% wheat germ oil;
Skin softening agents - 10% sweet almond oil;
Absorption aids - 10% jojoba oil;
Tissue regenerating and protecting agents - 10% grape seed oil;
Preservatives - 4% thyme essential oil;
Thickening agents - 10% bee wax, 10% coconut oil, 5% guar gum, 5% palm oil and 2% borax.

An ointment produced according to the above preferred embodiment has been applied to the skin of a Psoriasis patient suffering from 90% affliction. The ointment has been applied three times each day during four days to the pateint's scalp and body. The patient reported complete disappearance of the psoriatic plaques, both on the scalp and on the legs, arms and back, itching was reduced by 70% and the tissues showed signs of healing.

## Claims

1. Cosmetic and dermatological composition for the treatment of psoriatic skin, comprising 5% - 15% quince seeds; 10% - 40% natural base cream; and 55% - 75% mixture of anti-oxidants, skin softening agents, absorption aids, tissue regenerating and protecting agents, preservatives and thickening agents.

2. The composition of claim 1, comprising 10% quince seeds; 20% natural base cream; and 70% mixture of anti-oxidants, skin softening agents, absorption aids, tissue regenerating and protecting agents, preservatives and thickening agents.

3. The composition of claim 1 or 2, wherein the anti-oxidants are selected from the group consisting of Vitamin E, wheat germ oil and Safflower oil.

4. The composition of claim 1, 2 or 3 wherein the skin softening agents are selected from the group consisting of sweet almond oil and sesame oil.

5. The composition of any one preceding claim, wherein the absorption aids are selected from the group consisting of jojoba oil and vegetable squalene.

6. The composition of any one preceding claim, wherein the tissue regenerating and protecting agents are selected from the group consisting of grape seed oil and sunflower oil.

7. The composition of any one preceding clainm, wherein the preservatives are selected from the group consisting of paraben, tea tree essential oil, thyme essential oil, grapefruit seed extract and D-Alpha Tocopherol Acetate.

8. The composition of any one preceding claim, wherein the thickening agents are selected from the group consisting of bee wax, aloevera, medicinal vaseline, coconut oil, guar gum, palm oil and borax.

9. A method of producing a composition for the treatment of psoriatic skin, comprising the steps of:
soaking quince seeds in water, whereby jelly-like consistency is attained, comprising 5% - 15% of the composition weight;
stirring natural base cream comprising 10% - 40% of the composition weight into the quince seeds jelly; and
adding a mixture of anti-oxidants, skin softening agents, absorption aids, tissue regenerating and protecting agents, preservatives and thickening agents, said mixture comprising 55% - 75% of the composition weight.

10. The method of claim 9, wherein the quince seeds jelly comprises 10% of the composition weight; the natural base cream comprises 20% of the composition weight; and the mixture of anti-oxidants, skin softening agents, absorption aids, tissue regenerating and protecting agents, preservatives and thickening agents comprises 70% of the composition weight.

11. The method of claim 9 or 10, wherein the anti-oxidants are selected from the group consisting of Vitamin E, wheat germ oil and Safflower oil.

12. The method of claim 9, 10 or 11, wherein the skin softening agents are selected from the group consisting of sweet almond oil and sesame oil.

13. The method of any one of claims 9 to 12, wherein the absorption aids are selected from the group consisting of jojoba oil and vegetable squalene.

14. The method of cany one of claims 9 to 13, wherein the tissue regenerating and protecting agents are selected from the group consisting of grape seed oil and sunflower oil.

15. The method of any one of claims 9 to 14, wherein the preservatives are selected from the group consisting of paraben, tea tree essential oil, thyme essential oil, grapefruit seed extract and D-Alpha Tocopherol Acetate.

16. The method of any one of claims 9 to 15, wherein the thickening agents are selected from the group consisting of bee wax, aloevera, medicinal vaseline, coconut oil, guar gum, palm oil and borax.
